# EUROPÄISCHE PATENTSCHRIFT

(11) **EP 1 308 150 B1**
(45) Veröffentlichungstag und Bekanntmachung des Hinweises auf die Patenterteilung: **04.02.2004**
(21) Anmeldenummer: 01125978.5
(22) Anmeldetag: 31.10.2001
(51) Int. Cl.: A61K 7/06

(54) **Haarstylingstift auf Basis von 3 Polyethylenglykolen verschiedener Molekulargewichte**
Hairstyling stick comprising 3 polyethylene glycols of different molecular weights
Bâton pour le coiffage comprenant 3 polyéthylène glycols avec des poids moléculaires différents

(43) Veröffentlichungstag der Anmeldung: 07.05.2003
(73) Patentinhaber: Wella Aktiengesellschaft, 64274 Darmstadt (DE)
(72) Erfinder: Birkel, Susanne, 64285 Darmstadt (DE); Franzke, Michael, 64380 Rossdorf (DE); Hannich, Manuela, 63329 Egelsbach (DE); Stein, Bernd, 63768 Hösbach (DE); Wendel, Harald, 64372 Ober-Ramstadt (DE)

(56) Entgegenhaltungen:
- EP-A- 1 053 742
- US-A- 4 938 954
- US-A- 5 500 218
- US-A- 5 690 924
- US-B1- 6 241 978

## Beschreibung

Gegenstand der Erfindung ist ein Haarstylingstift (Styling Stick) mit einer Zusammensetzung von fester, wachsartiger Konsistenz enthaltend eine Kombination von drei Polyethylenglykolen unterschiedlicher Molekulargewichte.

Stylingwachs-Zusammensetzungen sind bekannte Produkte zur Haarbehandlung. Sie finden insbesondere Anwendung, um kurzes bis mittellanges Haar trendgerecht in Form zu bringen. Mit derartigen Produkten sind beispielsweise Frisurengestaltungen in Form von hochgestellten Ponypartien, gestriegelten Vorderköpfen, sowie sogenannte "Dirt-Look"- oder "Spiky Look"-Frisuren möglich. Auch lassen sich hiermit Konturen und Texture in der Frisur erzeugen. Herkömmliche Stylingwachs-Produkte werden üblicherweise in Tiegeln angeboten. Die Entnahme der Produktmasse erfolgt mit den Fingern. Das Wachs wird in den Handflächen verteilt, wobei es aufgrund der über die Hände übertragenen Körperwärme erweicht. Durch die Erweichung wird die Einarbeitung des ansonsten zu festen Wachses in das Haar ermöglicht. Nach Einarbeitung in das Haar kühlt das Wachs wieder ab, erhärtet dabei und die gestaltete Frisur erhält Stabilität und einen leichten Wet-Look.

Der Nachteil dieser Applikationsform ist, dass die Hände des Anwenders intensiv mit dem Wachs in Berührung kommen und anschließend gereinigt werden müssen. Die Aufgabe der vorliegenden Erfindung bestand darin, ein Produkt zu entwickeln, welches im wesentlichen die Produkteigenschaften von Tiegel-Wachsen aufweist, d.h. dem Haar Glanz, Pflege und Halt verleiht, dabei aber in einer anderen Applikationsform angewandt wird, bei der die Finger bzw. die Handflächen nicht benutzt werden müssen. Eine geeignete Applikationsform ist ein Haarstylingstift, im folgenden Styling Stick genannt. An die hierfür benötigte Zusammensetzung sind eine Reihe von Anforderungen zu stellen. Die Zusammensetzung darf nicht zu hart sein und muss sich auch ohne durch Erwärmung hervorgerufene Erweichung bei direkter Auftragung auf das Haar gut auf dem Haar verteilen lassen. Gleichzeitig darf die Zusammmensetzung nicht zu weich sein, da ansonsten der gewünschte Stand und Halt der erstellten Frisur nicht erreicht wird. Der Abrieb darf nicht zu gering und nicht zu stark sein und die Konsistenz darf nicht spröde sein. Ausserdem darf die Zusammensetzung nicht zu klebrig sein, da ansonsten Haare an der Stiftmasse haften bleiben, wenn der Stift bei der Anwendung über das Haar gestrichen wird.

Herkömmliche Stylingsticks sind in der Regel auf der Basis von hydrophoben Wachsen, Fetten und Ölen aufgebaut. Sie enthalten einen großen Anteil an hydrophoben Stoffen wie z.B. pflanzlichen oder tierischen Wachsen, Fettsäureestern, Fettalkoholen etc.. Derartige Produkte haben den Nachteil, dass sie schlecht aus dem Haar auswaschbar sind und eine relativ hohe Belastung des Haares bewirken.

Aus der EP 0 301 197 A1 ist ein Haarwachs bekannt, welches eine Kombination von hochmolekularem Polyethylenglykol (MG = 3000 bis 5000), oxethyliertem und hydriertem Rizinusöl und niedrigmolekularem Polyethylenglykol (MG = 100 bis 300) enthält. Es werden nur Tiegel-Produkte beschrieben, aber keine Sticks. Mit der in der EP 0 301 197 A1 beschriebenen Kombination von zwei Polyethylenglykolen stark unterschiedlichen Molekulargewichts ist die Herstellung von Haarstylingsticks mit völlig zufriedenstellenden Produkteigenschaften nicht möglich. Bei stark unterschiedlichem Molekulargewicht weisen die Sticks eine zu grobe und spröde Konsistenz auf. Außerdem kann es zu Inhomogenitäten der Wachsmasse nach dem Abkühlen kommen, was sich dadurch bemerkbar macht, dass der Stick innen weicher als aussen ist.

Aus Janistyn, Handbuch der Kosmetika und Riechstoffe, Band 3 (1973) Seite 325 ist eine Stangenpomade bestehend aus 60 Gew.% Polyethylenglykol 4000 und 40 Gew.% Ethylhexandiol bekannt. Mit einer derartig zusammengesetzten Stangenpomade behandelte Haare besitzen jedoch weder einen befriedigenden Glanz noch eine ausreichende Formstabilität oder ein befriedigendes Standvermögen. Zudem handelt es sich bei Ethylhexandiol um eine reizende Substanz, deren Einsatz in kosmetischen Produkten nach Möglichkeit vermieden werden sollte.

Es wurde nun gefunden, dass die Anforderungen erfüllt werden durch einen Haarstylingstift (Styling Stick) mit einer Zusammensetzung von fester, wachsartiger Konsistenz enthaltend eine Kombination von mindestens drei Polyethylenglykolen unterschiedlicher Molekulargewichte. Der Stick mit drei Polyethylenglykolen zeichnet sich gegenüber einer Kombination von nur zwei Polyethyenglykolen insbesondere durch eine größere Homogenität der Konsistenz aus.

Gegenstand der Erfindung ist daher ein Haarstylingstift mit einer festen, wachsartigen Zusammensetzung enthaltend
(A) mindestens ein Polyethylenglykol mit einem Molekulargewicht von 2500 bis 5000,
(B) mindestens ein Polyethylenglykol mit einem Molekulargewicht von 850 bis 1600 und
(C) mindestens ein Polyethylenglykol mit einem Molekulargewicht von 370 bis 800.
sowie gegebenenfalls Wasser, ein- oder mehrwertige C1-C5-Alkohole, Emulgatoren, Parfüm, Farbstoffe und/oder Perlglanzpigmente.

Die erfindungsgemäße Kombination von Polyethylenglykolen mit ganz bestimmten Molekulargewichten ist hervorragend dazu geeignet, um Haarstylingprodukte in Stiftform herzustellen. Das hochmolekulare Polyethylenglykol (A) ist bei Raumtemperatur (20-25°C) wachsartig fest. Es wird in Konzentrationen von vorzugsweise 30 bis 55 Gew.%, besonders bevorzugt von 35 bis 45 Gew.% eingesetzt. Das Molekulargewicht beträgt 2500 bis 5000 g/mol, vorzugsweise 2700 bis 3500 g/mol. Polyethylenglykole besitzen die allgemeine Formel H(OCH₂CH₂)ₙOH. Geeignete hochmolekulare Polyethylenglykole sind solche mit n = 57 bis 113, vorzugsweise mit n = 61 bis 79. Geeignete Polyethylenglykole haben die INCI-Bezeichnungen PEG-60 (n = 60), PEG-75 (n = 75), PEG-90 (n = 90) und PEG-100 (n = 100). Besonders bevorzugt sind PEG-60 und PEG-75. Handelsprodukte weisen in der Regel eine Molekulargewichtsverteilung auf. Geeignete Handelsprodukte sind z.B. Polyglykol 3000 mit einem Molekulargewicht von 2700 bis 3000 oder Polyglykol 4000 der Firma Clariant mit einem Molekulargewicht von 3700 bis 4500. Hierbei ist das Polyglykol 3000 vorteilhafter als das Polyglykol 4000, da es in der erfindungsgemäßen Kombination zu weicheren Sticks mit vermehrtem Abrieb führt.

Das Polyethylenglykol (B) mit mittlerem Molekulargewicht ist vorzugsweise bei Raumtemperatur (20-25°C) weichwachsartig mit einer Erstarrungstemperatur von etwa 30 bis 45°C. Es wird in Konzentrationen von vorzugsweise 15 bis 35 Gew.%, besonders bevorzugt von 20 bis 30 Gew.% eingesetzt. Das Molekulargewicht beträgt von etwa 850 bis etwa 1600 g/mol, vorzugsweise 850 bis 1200 g/mol. Polyethylenglykole besitzen die allgemeine Formel H(OCH₂CH₂)ₙOH. Geeignete Polyethylenglykole (B) mit mittlerem Molekulargewicht sind solche mit n = 19 bis 36, vorzugsweise mit n = 20 bis 24. Geeignete Polyethylenglykole haben die INCI-Bezeichnungen PEG-20 (n = 20) und PEG-32 (n = 32). Besonders bevorzugt ist PEG-20. Handelsprodukte weisen in der Regel eine Molekular gewichtsverteilung auf. Geeignete Handelsprodukte sind z.B. Polyglykol 1000 mit einem Molekulargewicht von 950 bis 1050 oder Polyglykol 1350 mit einem Molekulargewicht von 1300 bis 1400 oder Polyglykol 1500 mit einem Molekulargewicht von 1400 bis 1600 der Firma Clariant. Hierbei ist das Polyglykol 1000 am vorteilhaftesten.

Das niedrigmolekulare Polyethylenglykol (C) ist bei Raumtemperatur (20-25°C) vorzugsweise flüssig. Es wird in Konzentrationen von vorzugsweise 15 bis 35 Gew.%, besonders bevorzugt von 20 bis 30 Gew.% eingesetzt. Das Molekulargewicht beträgt 370 bis 800 g/mol, vorzugsweise 400 bis 640 g/mol. Geeignete niedrigmolekulare Polyethylenglykole sind solche der Formel H(OCH₂CH₂)ₙOH mit n = 8 bis 16, vorzugsweise mit n = 9 bis 14. Geeignete Polyethylenglykole haben die INCI-Bezeichnungen PEG-8 (n=8), PEG-9 (n = 9), PEG-10 (n = 10), PEG-12 (n = 12), PEG-14 (n = 14) und PEG-16 (n = 16). Besonders bevorzugt sind PEG-9, PEG-10, PEG-12 und PEG-14. Geeignete Handelsprodukte sind z.B. Polyglykol 400 mit einem Molekulargewicht von 380 bis 420 oder Polyglykol 600 der Firma Clariant mit einem Molekulargewicht von 570 bis 630. Hierbei hat sich das Polyglykol 600 als vorteilhafter als das Polyglykol 400 erwiesen.

Es hat sich gezeigt, dass Stylingsticks, die zwei Polyethylenglykole enthalten, eine schlechtere, gröbere und sprödere Konsistenz aufweisen, je weiter das Molekulargewicht der beiden Polyethylenglykole auseinanderliegt. Ein Vergleich einer erfindungsgemäßen Stick-Zusammensetzung mit einer Zusammensetzung entsprechend der EP 0 301 197, welche neben einem hochmolekularen ein niedrigmolekulares Polyethylenglykol (Polyglykol 200, molare Masse 190 bis 210 g/mol) enthielt, führte zu einem wesentlich gröberen und spröderen Stick. Diese beobachtete Sprödigkeit hängt vermutlich mit dem Kristallisationsverhalten der Gesamtzusammensetzung zusammen. Die Herstellung des Sticks erfolgt in der Regel in der Weise, dass die Komponenten bei einer solchen erhöhten Temperatur vermischt werden, bei der auch das hochmolekulare Polyethylenglykol flüssig vorliegt. Anschließend wird abgekühlt, was in der Regel mittels einer zusätzlichen äußeren Kühlung erfolgt. Hierbei erstarrt das hochmolekulare Polyethylenglykol, wobei die Kristallisation exotherm verläuft. Es hat sich gezeigt, dass die Auswahl des niedrigmolekularen Polyethylenglykols einen Einfluss auf das Kristallisationsverhalten hat. Je weiter die Molekulargewichte der eingesetzten Polyethylenglykole auseinanderliegen, umso gröber und spröder wird der Stick.

Andererseits soll das niedrigmolekulare Polyethylenglykol bei Raumtemperatur noch flüssig sein, da sonst die Produktleistungen hinsichtlich Glanz und Verteilbarkeit auf dem Haar beeinträchtigt sind. Sehr gut geeignet ist daher Polyglykol 600 mit einer molaren Masse von 570 bis 630 g/mol und einer Erstarrungstemperatur von 17 bis 22 °C, d.h. knapp unterhalb von Raumtemperatur.

Das Massenverhältnis von hochmolekularem Polyethylenglykol (A) zur Summe der beiden niedriger molekularen Polyethylenglykole (B) und (C) beträgt vorzugsweise von 1 : 0,9 bis 1 : 1,8, besonders bevorzugt von 1 : 1 bis 1 : 1,6. Das Massenverhältnis von Polyethylenglykol (B) zum Polyethylenglykol (C) beträgt vorzugsweise von 1 : 0,7 bis 1 : 1,3, besonders bevorzugt von 1 : 0,8 bis 1 : 1,2. Art und Mengen der Einsatzstoffe sind außerdem so gewählt, dass der Tropfpunkt des fertigen Sticks vorzugsweise bei mindestens 40°C, insbesondere im Bereich von 40 bis 60 °C, besonders bevorzugt im Bereich von 45 bis 55°C liegt.

Der erfindungsgemäße Stylingstick ermöglicht aufgrund der wachsartigen Konsistenz und seiner kohäsiven Eigenschaften eine individuelle Frisurengestaltung sowie die gezielte Behandlung einzelner Haarsträhnen. Die Produktmasse ist leicht auf dem Haar verteilbar, ohne dass die Hände oder Finger mit der Produktmasse in Berührung kommen. Das behandelte Haar zeichnet sich durch einen ausgeprägten Glanz sowie durch eine hohe Formstabilität der erstellten Frisur aus. Das Haar wird dabei nicht übermäßig belastet und die Produktmasse ist leicht auswaschbar.

Zusätzlich zu den vorstehend genannten Inhaltsstoffen kann die Stick-Zusammensetzung weitere Zusatzstoffe enthalten:
- Lösungsmittel wie Wasser oder ein- oder mehrwertige C1- bis C4-Alkohole, insbesondere Ethanol, Propanol, Glycerine oder Glykole in einer Menge bis zu 10 Gew.%, vorzugsweise 0,1 bis 8 Gew.%
- Kosmetische Farbstoffe in einer Menge bis zu 6 Gew.%, vorzugsweise 0,1 bis 4 Gew.%, z.B. C.I. Pigment Red 4 (C.I. 12 085), C.I. Pigment Green (C.I. 74 260), und/oder C.I. Vat Blue 4 (C.I. 69 800)
- Perlglanzpigmente in einer Menge von bis zu 25 Gew.%, vorzugsweise 1 bis 20 Gew.%, z.B. solche auf Titandioxid/Mica-Basis
- Emulgatoren in einer Menge von bis zu 25 Gew.%, vorzugsweise 0,1 bis 18 Gew.%, z.B. hydriertes und ethoxyliertes Rizinusöl
- Parfüm- und Duftstoffe in einer Menge von bis zu 2 Gew.%, vorzugsweise 0,01 bis 1 Gew.%
- Konservierungsmittel in einer Menge von bis zu 1 Gew.%, vorzugsweise 0,01 bis 0,5 Gew.%, insbesondere Parahydroxybenzoesäureester, Benzoesäure, Salicylsäure, Sorbinsäure, Mandelsäure, Polyhexamethylenbiguanid-hydrochlorid oder Isothiazolinonderivate, von denen sich die Sorbinsäure als besonders geeignet erwiesen hat
- Hydrophobe Wachse, Fette oder Öle, z.B. pflanzliche, tierische oder mineralische Wachse wie Ozokerit, Fettsäureester, Fettalkohole in einer Menge von bis zu 5 Gew.%, vorzugsweise 0,1 bis 4 Gew.%
- Filmbildende Polymere wie z.B. Polyvinylpyrrolidon oder Vinylpyrrolidon/Vinylacetat Copolymere in einer Menge von bis zu 5 Gew.%, vorzugsweise 0,1 bis 4 Gew.%
- Haarpflegende Zusätze wie z.B. Betain in einer Menge von bis zu 5 Gew.%, vorzugsweise 0,01 bis 4 Gew.%.

Die Herstellung der Stylingsticks erfolgt, indem die Komponenten bei einer erhöhten Temperatur, bei der das hochmolekulare Polyethylenglykol (A) in flüssiger Form vorliegt (z.B. ca. 65 bis 90°C), unter Rühren aufgeschmolzen und miteinander vermischt werden. Anschließend wird die homogene Schmelze gegebenenfalls etwas abgekühlt und in noch flüssiger Form in Verpackungen mit der gewünschten Stick-Form (Stickhülsen) abgefüllt. Unter weiterer Abkühlung, vorzugsweise mit externer Kühlung (z.B. 0-10°C) erstarrt die Produktmasse. Bei schneller Abkühlung mit zusätzlicher externer Kühlung erhalten die Sticks eine größere Festigkeit als bei langsamerer Abkühlung ohne externe Kühlung.

Als Verpackungsmaterial können die üblichen, für kosmetische Sticks verwendeten Materialien eingesetzt werden, insbesondere Kunststoffe oder Metalle. Der geometrischen Form der Sticks sind keine Grenzen gesetzt. Es kann sich um eine zylindrische Form mit runder oder ovale Grundfläche aber auch um Formen mit eckigen Grundflächen handeln.

Die Applikation des Wachses in Stick-Form ist sehr einfach. Der Stift wird über das Haar gezogen und hinterläßt so die Produktmasse. Das Haar erhält dadurch Glanz, Texture und/oder Halt.

Die nachfolgenden Beispiele sollen den Gegenstand der Erfindung näher erläutern.

### Beispiele

Die Sticks mit den nachfolgenden Zusammensetzungen werden hergestellt, indem die Komponenten bei 85°C unter Rühren und Aufschmelzen vermischt werden. Nach Abkühlen auf 65-70°C werden die Massen in Stickhülsen abgefüllt. Unter Kühlung (4°C) erstarren die Massen in den Hülsen.

| **Beispiel 1** Styling Stick | |
|---|---|
| 28,5 g | PEG-12 (Polyglykol 600) |
| 28,5 g | PEG-20 (Polyglykol 1000) |
| 43,0 g | PEG-60 (Polyglykol 3000) |

| **Beispiel 2** Styling Stick | |
|---|---|
| 28,1 g | PEG-12 (Polyglykol 600) |
| 28,0 g | PEG-20 (Polyglykol 1000) |
| 43,0 g | PEG-60 (Polyglykol 3000) |
| 0,5 g | Wasser |
| 0,2 g | Parfüm |
| 0,1 g | Betain |
| 0,1 g | Glycerin |

| **Beispiel 3** Styling Stick | |
|---|---|
| 29,4 g | PEG-12 (Polyglykol 600) |
| 29,3 g | PEG-20 (Polyglykol 1000) |
| 40,0 g | PEG-60 (Polyglykol 3000) |
| 0,5 g | Wasser |
| 0,3 g | PEG-25 Hydrogenated Castor Oil |
| 0,3 g | Parfüm |
| 0,1 g | Betain |
| 0,1 g | Ozokerit |

| **Beispiel 4** Styling Stick | |
|---|---|
| 27,75 g | PEG-12 (Polyglykol 600) |
| 27,7 g | PEG-20 (Polyglykol 1000) |
| 43,0 g | PEG-60 (Polyglykol 3000) |
| 0 , 5 g | Wasser |
| 0,3 g | PEG-25 Hydrogenated Castor Oil |
| 0 , 3 g | Parfüm |
| 0,25 g | Sorbinsäure |
| 0,1 g | Betain |
| 0,1 g | Ozokerit |

## Patentansprüche

1. Haarstylingstift mit einer festen, wachsartigen Zusammensetzung enthaltend
(A) mindestens ein Polyethylenglykol mit einem Molekulargewicht von 2500 bis 5000,
(B) mindestens ein Polyethylenglykol mit einem Molekulargewicht von 850 bis 1600 und
(C) mindestens ein Polyethylenglykol mit einem Molekulargewicht von 370 bis 800.

2. Haarstylingstift nach Anspruch 1, **dadurch gekennzeichnet, dass** das niedrigermolekulare Polyethylenglykol (C) bei Raumtemperatur (20-25°C) flüssig ist.

3. Haarstylingstift nach einem der vorhergehenden Ansprüche, **dadurch gekennzeichnet, dass** das Polyethylenglykol (A) in einer Menge von 30 bis 55 Gew.%, das Polyethylenglykol (B) in einer Menge von 15 bis 35 Gew.% und das Polyethylenglykol (C) in einer Menge von 15 bis 35 Gew.% vorliegt.

4. Haarstylingstift nach einem der vorhergehenden Ansprüche, **dadurch gekennzeichnet, dass** das Mengenverhältnis des Polyethylenglykols (A) zur Gesamtmenge der Polyethylenglykole (B) und (C) von 1 : 0,9 bis 1 : 1,8 beträgt.

5. Haarstylingstift nach einem der vorhergehenden Ansprüche, **dadurch gekennzeichnet, dass** das Mengenverhältnis des Polyethylenglykols (B) zum Polyethylenglykol (C) von 1 : 0,7 bis 1 : 1,3 beträgt.

6. Haarstylingstift nach einem der vorhergehenden Ansprüche, **dadurch gekennzeichnet, dass** das Polyethylenglykol (A) ein Molekulargewicht im Bereich von 2700 bis 3500 g/mol aufweist.

7. Haarstylingstift nach einem der vorhergehenden Ansprüche, **dadurch gekennzeichnet**, dass-das Polyethylenglykol (B) ein Molekulargewicht im Bereich von 850 bis 1200 g/mol aufweist.

8. Haarstylingstift nach einem der vorhergehenden Ansprüche, **dadurch gekennzeichnet, dass** das Polyethylenglykol (C) ein Molekulargewicht im Bereich von 400 bis 640 g/mol aufweist.

9. Haarstylingstift nach einem der vorhergehenden Ansprüche, **dadurch gekennzeichnet, dass** mindestens ein weiterer Zusatzstoff enthalten ist, ausgewählt aus Wasser, ein- oder mehrwertigen C1- bis C4-Alkoholen, kosmetischen Farbstoffen, Perlglanzpigmenten, Emulgatoren, Parfüm- und Duftstoffen, Konservierungsmitteln, hydrophoben Wachsen, Fetten und Ölen, filmbildenden Polymeren und haarpflegenden Zusätzen.

10. Haarstylingstift nach einem der vorhergehenden Ansprüche, **gekennzeichnet durch** einen Gehalt an
(A) 30 bis 55 Gew.% eines Polyethylenglykols mit einem Molekulargewicht von 2700 bis 3500,
(B) 15 bis 35 Gew.% eines Polyethylenglykols mit einem Molekulargewicht von 850 bis 1200 und
(C) 15 bis 35 Gew.% eines Polyethylenglykols mit einem Molekulargewicht von 400 bis 640.

## Claims

1. Hairstyling stick with a solid, wax-like composition comprising
(A) at least one polyethylene glycol with a molecular weight of from 2 500 to 5 000,
(B) at least one polyethylene glycol with a molecular weight of from 850 to 1 600 and
(C) at least one polyethylene glycol with a molecular weight of from 370 to 800.

2. Hairstyling stick according to Claim 1, **characterized in that** the lower-molecular weight polyethylene glycol (C) is liquid at room temperature (20-25°C).

3. Hairstyling stick according to one of the preceding claims, **characterized in that** the polyethylene glycol (A) is present in an amount of from 30 to 55% by weight, the polyethylene glycol (B) is present in an amount of from 15 to 35% by weight and the polyethylene glycol (C) is present in an amount of from 15 to 35% by weight.

4. Hairstyling stick according to one of the preceding claims, **characterized in that** the quantitative ratio of the polyethylene glycol (A) to the total amount of the polyethylene glycols (B) and (C) is from 1:0.9 to 1:1.8.

5. Hairstyling stick according to one of the preceding claims, **characterized in that** the quantitative ratio of the polyethylene glycol (B) to the polyethylene glycol (C) is from 1:0.7 to 1:1.3.

6. Hairstyling stick according to one of the preceding claims, **characterized in that** the polyethylene glycol (A) has a molecular weight in the range from 2 700 to 3 500 g/mol.

7. Hairstyling stick according to one of the preceding claims, **characterized in that** the polyethylene glycol (B) has a molecular weight in the range from 850 to 1 200 g/mol.

8. Hairstyling stick according to one of the preceding claims, **characterized in that** the polyethylene glycol (C) has a molecular weight in the range from 400 to 640 g/mol.

9. Hairstyling stick according to one of the preceding claims, **characterized in that** at least one further additive is present, chosen from water, mono- or polyhydric C₁- to C₄-alcohols, cosmetic dyes, pearlescent pigments, emulsifiers, perfumes and fragrances, preservatives, hydrophobic waxes, fats and oils, film-forming polymers and haircare additives.

10. Hairstyling stick according to one of the preceding claims, **characterized by** a content of
(A) 30 to 55% by weight of a polyethylene glycol with a molecular weight of from 2 700 to 3 500,
(B) 15 to 35% by weight of a polyethylene glycol with a molecular weight of 850 to 1 200 and
(C) 15 to 35% by weight of a polyethylene glycol with a molecular weight of from 400 to 640.

## Revendications

1. Bâton de coiffure avec une composition solide cireuse comprenant
(A) au moins un polyéthylèneglycol présentant un poids moléculaire de 2 500 à 5 000,
(B) au moins un polyéthylèneglycol présentant un poids moléculaire de 850 à 1 600, et
(C) au moins un polyéthylèneglycol présentant un poids moléculaire de 370 à 800.

2. Bâton de coiffure selon la revendication 1, **caractérisé en ce que** le polyéthylèneglycol à plus faible poids moléculaire (C) est liquide à la température ambiante (de 20 à 25°C).

3. Bâton de coiffure selon l'une quelconque des revendications précédentes, **caractérisé en ce que** le polyéthylèneglycol (A) est présent en une quantité de 30 à 55 % en poids, le polyéthylèneglycol (B) est présent en une quantité de 15 à 35 % en poids et le polyéthylèneglycol (C) est présent en une quantité de 15 à 35 % en poids.

4. Bâton de coiffure selon l'une quelconque des revendications précédentes, **caractérisé en ce que** le rapport de quantité entre le polyéthylèneglycol (A) et la quantité totale des polyéthylèneglycols (B) et (C) est de 1:0,9 à 1:1,8.

5. Bâton de coiffure selon l'une quelconque des revendications précédentes, **caractérisé en ce que** le rapport de quantité entre le poléthylèneglycol (B) et le polyéthylèneglycol (C) est de 1:0,7 à 1:1,3.

6. Bâton de coiffure selon l'une quelconque des revendications précédentes, **caractérisé en ce que** le polyéthylèneglycol (A) présente un poids moléculaire dans la plage de 2 700 à 3 500 g/mol.

7. Bâton de coiffure selon l'une quelconque des revendications précédentes, **caractérisé en ce que** le polyéthylèneglycol (B) présente un poids moléculaire dans la plage de 850 à 1 200 g/mol.

8. Bâton de coiffure selon l'une quelconque des revendications précédentes, **caractérisé en ce que** le polyéthylèneglycol (C) présente un poids moléculaire dans la plage de 400 à 640 g/mol.

9. Bâton de coiffure selon l'une quelconque des revendications précédentes, **caractérisé en ce qu'**au moins un additif supplémentaire est présent, choisi parmi l'eau, des alcools en C1 à C4 monohydriques ou polyhydriques, des colorants cosmétiques, des pigments nacrés, des émulsifiants, des parfums et des fragrances, des conservateurs, des cires, des graisses et des huiles hydrophobes, des polymères filmogènes et des additifs de soin des cheveux.

10. Bâton de coiffure selon l'une quelconque des revendications précédentes, **caractérisé par** une teneur
(A) de 30 à 55 % en poids d'un polyéthylèneglycol présentant un poids moléculaire de 2 700 à 3 500,
(B) de 15 à 35 % en poids d'un polyéthylèneglycol présentant un poids moléculaire de 850 à 1 200, et
(C) de 15 à 35 % en poids d'un polyéthylèneglycol présentant un poids moléculaire de 400 à 640.
